# EUROPEAN PATENT APPLICATION

(11) **EP 2 011 903 A2**
(43) Date of publication of application: **07.01.2009**
(21) Application number: 08252311.9
(22) Date of filing: 05.07.2008
(51) Int. Cl.: C23F 1/16, C23F 1/26, C23F 1/44

(54) **Etching solution and method of its manufacturing as well as method of etching metal surfaces and microtextured implants made using such a method**

(30) Priority: 06.07.2007 US 958492 P; 01.07.2008 US 165773 P
(71) Applicant: DePuy Products, Inc., Warsaw, IN 46581 (US)
(72) Inventor: Tong, Weidong, Warsaw IN, 46580 (US); Salvati, Lawrence, Goshen IN, 46526 (US)
(74) Representative: Belcher, Simon James

(57) **Abstract**

A process for etching a metal such as a cobalt-chromium-molybdenum alloy involves contacting the metal with a solution comprising hydrogen chloride and a persulphate salt; in which the solution has a hydrogen chloride concentration of about 3 to about 11.7 moles/litre and a molar ratio of hydrogen chloride to persulphate salt of about 4:1 to about 134:1, in which the persulphate salt is dissolved in the solution with the use of heat input.

## Description

The present invention concerns methods for etching metal surfaces with a solution comprising hydrogen chloride and a persulphate salt and microtextured implants made using such a method.

There are a number of design criteria which have long been sought for segmental bone replacement implants including (1) the implant should last the lifetime of the patient without losing function or initiating any adverse process response; (2) the implant should restore the normal function of the bone in which it is implanted; and (3) the implant should be capable of being produced on a commercial scale. In order to satisfy these criteria, not only should the implant support the imposed load, often of a fluctuating nature, but the interface between the implant and the bone should also withstand the load requirement.

A plastic cement such as polymethyl methacrylate is often used to affix an implant to bone as well as to improve the fit between the implant and the bone. Implants also have been provided with porous coatings which mate with the bone and invite bone ingrowth such that, after a period of time, the prosthesis becomes integrated into the bone structure. Typical of such coatings are those disclosed in US-3855638, US-4206516, US-4156943 and US-4612160.

Indeed, the effectiveness of an orthopaedic implant often depends upon the presence of an irregular surface on the implant, into which the bone may grow to create a natural joinder between the bone and the implant. Several techniques have been used to create implants with irregular surfaces. Grit blasting is one surface roughening technique, but grit blasting can cause significant changes to the surface topography by damaging the metal elements (e.g., beads) which are bonded on the surface layer of the substrate. Other mechanical roughening techniques, such as scratching or burr grinding, have also been used. These techniques can also present drawbacks, including distortion of the substrate, removal of excess material, inability or difficulty to roughen certain surfaces, and inconsistent surface roughening.

Ceramic coatings have also been used to good effect and often are particularly desirable because of the affinity between bone and ceramic materials such as alumina (Al₂O₃). Typical of such coatings are those disclosed in US-4145764 and US-4483678 which are particularly concerned with dental implants, and US-4309488 and US-4846837 which more broadly disclose implantable bone replacement material for use throughout the body.

Other work has utilized highly convoluted surfaces on the implant. US-5368881 and US-5658333 show use of non-spherical powder to produce a roughened surface for prosthesis.

Some metal implants are fabricated from surgical grade cobalt-chromium-molybdenum (CoCrMo) alloys because these alloys show good corrosion and wear resistance. CoCrMo surfaces of such alloys can be grit-blasted with grit-medium such as alumina and/or glass beads. Mechanical roughening by grit blasting, however, is a line of sight technology and can not roughen the hidden side of a surface, such as within the porous surface structure of an implant (for example when the surface structure is provided by bonded metal beads as in implants sold under the trade mark Porocoat in which beads are sintered). In addition, grit medium may be embedded in between the beads or leave residue on metal surface.

Electrochemical etching has been used with hydrogen chloride (HCl) to reveal the microstructure (carbides, grain boundary impurity) of CoCrMo alloys. Electrochemical etching, however, requires the electric current to pass through the etched surface. This method is found to generate bead neck cracking or preferential etching of the connection between sintered metal beads connection without little satisfaction of achieving microtexture on the bead surface.

Chemical etching often involves using toxic reagents, such as methanol, or a strong acid at a high temperature (as high as 80°C, for example), or strong acid mixtures. Chemical etching, even under these stringent conditions, can require several hours to several days to roughen a surface. Some methods of chemical etching also present the potential for preferential etching on grain boundaries, which can reduce the mechanical properties of the implants. Chemical etching, for example, using 80% methanol and 20% fuming HCl (37%) at high temperature (70°C) is generally recognized as presenting safety issues (Ferrari, M; Cagidiaco, M.C; Boracchini, A; Bertelli, E. The Journal of Prosthetic Dentistry, 1989, 62(5): p516-521).

Accordingly, there remains a need for improved and reliable methods to form an effective textured surface on a metal or metal alloy substrate. There is also a need for orthopaedic implants having a surface texture.

In one aspect, the present invention concerns solutions that comprise hydrogen chloride and a persulphate salt; wherein the solution has a hydrogen chloride concentration of about 3 to about 11.7 moles/liter, a molar ratio of hydrogen chloride to persulphate salt of about 4:1 to about 134:1 where the persulphate salt is dissolved in the solution with the use of heat input. Some embodiments additionally comprise ferrous chloride which can be present in a concentration of 0 to 3.5 M. In some solutions, the persulphate salt is ammonium persulphate and preferably is present in a concentration of 1 g to 106 g or, in some embodiments, 1 to 32 g/100 ml of solution.

The invention also provides methods comprising the steps of:
- providing a first solution comprising hydrogen chloride, wherein the hydrogen chloride is present at a concentration of about 6 moles/liter to about 12 moles/liter;
- combining the first solution with a second solution comprising a persulphate salt in water to produce a third solution; wherein the concentration of the persulphate salt in the second solution is 1 to 106 grams per 100 ml of water and controlling the temperature from 15°C to 100°C during dissolution, and the volume ratio of the first solution to second solution is about 3:5 to about 30:1.

In some solutions, the concentration of hydrogen chloride in the first solution is from 6 to 8 moles per litre. In certain embodiments, the first solution is formed at ambient temperature.

Preferably, the volume ratio of the first solution to the second solution is about 3:5 to about 30:1 and/or the persulphate salt is ammonium persulphate or potassium persulphate.

In certain preferred embodiments, the second solution is prepared by contacting ammonium persulphate with water under heating conditions to maintain the temperature at 15 to 100°C. In some preferred embodiments, the second solution is at a temperature of 15 to 100°C when it is combined with the first solution.

The methods of the invention can further comprise contacting the third solution with a metal object for a time sufficient to etch the metal. Certain methods further comprise removing the metal object from the third solution and rinsing the metal object with water. In these embodiments, the metal can be contacted with the third solution for at least one second, preferably at least 2 minutes. Preferably, the metal is contacted with the third solution for not more than about 60 minutes. Preferred metal objects include those comprising at least one of cobalt, chromium, and molybdenum, such as wrought CoCrMo.

In a preferred embodiment, the metal object is contacted with an acid solution prior to being contacted with the etching solution (third solution, for example). The metal object can optionally be rinsed between the various treatment steps. The metal object can also be dried after any of the treatment and rinsing steps.

Embodiments of the invention are described below by way of example with reference to the accompanying drawings, in which:
Figure 1 illustrates the etch power of a spectrum of etch recipes using 6N and 7N HCl.
Figure 2 shows scanning electron microscope (SEM) images of etched ASTM E1534 wrought CoCrMo disks.
Figure 3 shows SEM images of an etched LCS knee femoral CoCrMo Porocoat (DePuy Cork) implants.
Figure 4 shows SEM images of etched knee type Porocoat (CoCrMo) using a second solution prepared with and without controlled heat input.
Figure 5 shows temperature and pH profiles of a second solution prepared at room condition without controlled temperature (A) and at 45°C (B).
Figure 6 shows Porocoat^{®} beads which were etched. Each bead surface overlaid with craters ranging from 0.5 µm to 2 µm in sizes (B).

In one aspect, the invention describes methods for chemically etching metal objects, such as CoCrMo (wrought and cast alloys, for example), in order to form microtextured surface. Microtextured metal surfaces have been shown to improve the bone coverage as well as the mechanical aspect of fixation.

Certain methods of the invention use heat activated saturated ammonium persulphate as the oxidant. Application of heat during preparing oxidant (ammonium persulphate) solution (second solution) is believed to enable the formation of free radicals, higher solubility and higher thermal energy during etch reaction, all of which help achieve repeatable and uniform etch microtexture at an economic cost and safe condition. In some embodiments, the temperature of the second solution is at least 15°C, preferably at least 20°C, more preferably at least 30°C. The temperature of the second solution can be less than 100°C, preferably less than 85°C, more preferably less than 60°C, especially less than 50°C. In some embodiments, the second solution can be combined with the first solution at the same temperature range as used for the formation of the second solution.

In some aspects, the disclosed invention offers methods to achieve repeatable and uniform microtexture on a CoCrMo flat or bonded bead surface at an economic cost and safe condition (reduced acid concentration, and no pre-heat or external heat during etch process).

The first solution ("the acids mix") can be prepared by dissolving FeCl₂ in hydrochloric acid. In some embodiments, the hydrochloric acid (6 to 12N) can optionally then be combined with H₃PO₄. In some embodiments, the H₃PO₄ is added at ambient temperature. Typically, the FeCl₂ is added at a concentration of 20 to 1500 ppm in the acids mix. Hydrochloric acid is available commercially at various concentrations including 12 N. Phosphoric acid, commercially available at various concentrations including 85 wt % acid. In some embodiments, the acids are used without further purification.

The second solution can be prepared by dissolution of a persulphate salt at controlled temperature (15 to 100 ° C). Generally, the second solution contains 1 to 106 g of persulphate salt and 100 ml H₂O. In some embodiments, the persulphate salt is present at 10 to 40 g/100 ml of water. In one embodiment, the second solution contains 32 g ammonium persulphate and 100 ml H₂O. Persulphate salts, such as ammonium persulphate, are commercially available. The salt can be used without further purification if desired.

The water used to make the solutions described herein can be done in RO water.
Heat input (by controlled temperature) during dissolution of ammonium persulphate (oxidant) allows higher solubility than room temperature preparation without adding additional amount of water. The higher concentration of ammonium persulphate permits etching to occur at reduced acid (proton) concentration. While not wanting to be bound by theory, it is believed that the heat input during dissolution of ammonium persulphate produces persulphate free radical (·SO₄⁻), which is a stronger oxidising agent than persulphate ions (S₂O₄²⁻). This is believed to help achieve uniform microtexture on metal (CoCrMo, for example) bead surface.

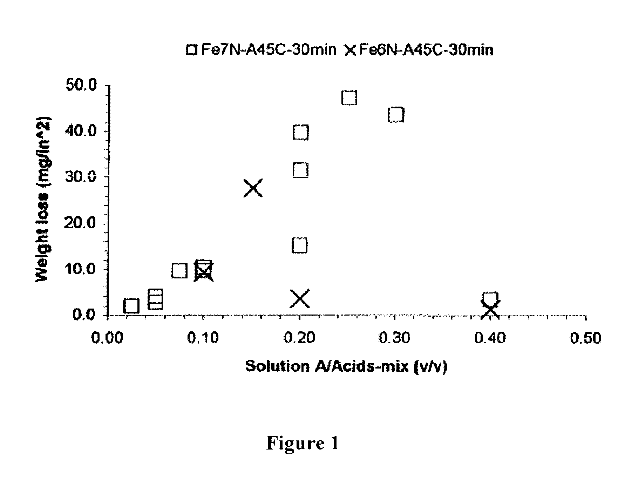

Oxidant (e.g., persulphate compound) is added to aid the etch process. It was discovered, however, that more oxidant does not always result in higher etch power. Too much oxidant can actually inhibit the etch process. A unique relationship between the proportion of acids mix and oxidant is discovered and established herein. See Figure 1. It is also noted that two equivalent recipes can be used to etch CoCrMo to achieve the same etch power and that the recipe with higher second solution to acids ratio can result in more heat transfer to acids due to larger amount of heated second solution that is utilized.

Metal objects according to the invention generally comprise at least one of cobalt, chromium, and molybdenum. Some objects comprise each of cobalt, chromium, and molybdenum. Certain objects are made of wrought CoCrMo. In some embodiments, the objects are metal disks or beads.

In one embodiment, the metal alloy is a cobalt-chromium alloy of the ASTM type F-75 (Standard Specification for Cobalt-28 Chromium-6 Molybdenum Alloy Castings and Casting Alloy for Surgical Implants, UNS R30075, Designation F75-01). In another embodiment, the metal alloy is a cobalt-chromium alloy of the ASTM type F-1537 (Standard Specification for Wrought Cobalt-28 Chromium-6 Molybdenum Alloys for Surgical Implants, UNS R31537, UNS R31538, and UNS R31539, Designation F1537-00). Exemplary materials typically contain chromium at about 26-30%, molybdenum at about 5 to 7% and a balance of cobalt. Another example of a chromium-containing alloy that can be used is stainless steel. In addition to chromium-containing alloys, other examples of metals that can be used are those which are stable in the body, and include, but are not limited to, titanium and titanium alloys. The shape of the implant will be selected according to its intended application.

The biomedical implant upon which an etched surface can be formed can be a metal body that has a coating of metallic elements adhered to at least a portion of the outer surface of the metal body. The metallic elements may form a three-dimensional porous surface geometry on the surface of the metallic biomedical implant. At least a portion of the metallic elements are interconnected to form pores between adjacent metallic elements (i.e. interstitial pores). These interstitial pores can range in size from about 10 µm to about 200 µm, and in some cases up to 750 µm. Such implants with coatings of metallic elements are referred to herein as "porous coated," and the coating of metallic elements is referred to as a "bonded bead", an example thereof being the coating that is provided on the surface of an implant by sintering, on products sold by DePuy Orthopaedics Inc under the trade mark Porocoat.

The metallic elements forming the porous coating can be provided in any suitable form. Generally, the metallic elements comprise metallic particles, metallic fibres, metallic wires, or combinations thereof. The metallic elements can be arranged in a predetermined pattern. For instance, a plurality of metallic fibres or wires can be arranged to form a mesh, which can be adhered to the outer surface of the metal body. In a preferred embodiment, the metallic elements comprise metallic particles. More preferably, the metallic particles comprise substantially spherical metallic beads. These metallic particles or beads can be of any suitable size. Typically, the size of the metallic particles or metallic beads is from about 40 µm to several millimetres.

The metal object (CoCrMo disks, for example) can be pre-warmed to be above room temperature (for example, using hot water). The acids mix can be added in the etch container with the metal object. The second solution is then added to the acids mix. The second solution can be added in the etch container at a range of (acids-mix):(second solution) ratio (v/v) of from 2.5 to 100.

One potential advantage of using a hot second solution is that it transfers heat to the acids when mixed and thus heats the acids in a safe fashion without preheating the acids.

Typically, the etch process lasts 2 to 60 minutes. In some embodiments, the methods generate 3-dimensional uniform microtextured topography on CoCrMo bonded beads (0.1 to 10 pits in diameter on bead surface).

In some preferred embodiments, (CoCrMo, for example) is presoaked in acid before combining the first solution with the second persulphate salt solution. In certain embodiments, pre-soak of the metal in acid allows for better control of the etch process due to the immediate reaction of persulphate salt with proton (our results indicate immediate drop of [H] in etch solution). In some embodiments, the speed of the etch proceeds faster with pre-soaking the metal in hydrogen chloride solution.

In some embodiments, the presoaking with acid is carried out over a minimum of 30 seconds. In certain embodiments, the proton concentration of the presoak solution is about 1 to about 12 moles/litre. In some embodiments, the proton concentration is about 3 to about 12, about 4 to about 10 or about 6 to about 9 moles/litre.

Useful acids for the presoak include hydrogen halide acids and mixtures of hydrogen halide acids and oxyacids. In some embodiments, the presoak solution comprises hydrochloric acid at a concentration in the range of about 1 to 12 moles/litre. In certain embodiments, an oxyacid can be present. One suitable oxyacid is phosphoric acid, which can be present at a concentration in the range of about 0.01 moles/litre to 14 moles/litre. Various other chemicals, such as chlorine containing compounds can be used in the etching solution. Typical concentrations of such compounds is 0.01 to 2 moles/litre.

Examples of hydrogen halide acids include, but are not limited to hydrogen fluoride, hydrogen chloride (hydrochloric acid), hydrogen bromide, and hydrogen iodide. In one embodiment, the hydrogen halide is hydrochloric acid at a concentration in the range of about 1M to 12M; about 3.5M to 8M; and about 4.6M.

Oxyacids have the general formula HₐX_{b}O_{c}, where "a" represents the number of hydrogen atoms, "X" represents an element other than hydrogen or oxygen, "b" represents the number of "X" atoms, and "c" represents the number of oxygen atoms. Examples of oxyacids include, but are not limited to, nitric acid, (HNO₃), sulphuric acid, (H₂SO₄), and phosphoric acid, (H₃PO₄). In one embodiment, the oxyacid is phosphorous containing acid at a concentration in the range of about 0.01 M to 14 M; about 4 M to 8 M; and about 5.6 M. Examples of phosphorous-containing oxyacids include, but are not limited to, ortho-phosphoric acid, peroxomonophosphoric acid, diphosphoric acid, peroxodiphosphoric acid, triphosphoric acid, hypophosphoric acid, polyphosphoric acid, isohypophosphoric acid, cyclo-trimetaphosphoric acid, phosphonic acid, cyclotetrametaphosphoric acid, diphosphonic acid, polymetaphosphoric acid, phosphinic acid, and anhydrous oxyacid.

A chlorine containing compound contains chlorine, which is typically present as a chloride. Most chlorides are salts that are formed either by direct union of chlorine with a metal or by reaction of hydrochloric acid (a water solution of hydrogen chloride) with a metal, a metal oxide, or an inorganic base. Examples of chlorine containing compounds include, but are not limited to, sodium chloride (NaCl), potassium chloride (KCl), calcium chloride (CaCl₂), ammonium chloride (NH₄Cl) and ferrous chloride (FeCl₂,), or mixtures thereof. In one embodiment, the chlorine containing compound has a concentration in the range of about 0.01 M to 2 M; about 0.6 M to 1.1 M; and about 0.9 M.

Before the etching process begins, the substrate surface can be cleaned using typical usual cleaning procedures, such as degreasing (e.g., either chemical, electrochemical or electrolytic). Alternatively, other chemical cleaning operations may be used, such as cleaning with an alkaline solution. The substrate surface may be degreased by ultrasonic cleaning in detergent, followed by ultrasonic cleaning in operating room water. The cleaned metal surface is then exposed to a suitable volume of etching solution in a container or bath. The volume of the etching solution depends on the surface area of the substrate for which etching is desired. In some instances, the entire surface of the implant will be etched, and thus the volume of the etching solution should be sufficient to cover the entire implant. In other applications, only a portion of the implant will be etched and only a desired portion of the implant need be exposed to the etching solution. One skilled in the art will readily appreciate the volume of etching solution that is required for a given etching procedure.

The following definitions are provided for the full understanding of terms used herein.

As used herein, the term "contact" or "contacting" means bringing together, either directly or indirectly, one composition or solution into physical proximity to another composition or solution. Contacting can involve two solutions, two neat compounds, or between a neat compound and a solution.

As used herein the term "mixture" means a solution, suspension, dispersion or emulsion. "Emulsion" refers to a mixture of two or more generally immiscible liquids, and is generally in the form of a colloid. "Suspension" or "dispersion" refers to a mixture, preferably finely divided, of two or more phases (solid and liquid, for example), which preferably can remain stable for extended periods of time.

The term "ambient temperature" is intended to mean room temperature. In some embodiments, this temperature is 20 to 25°C.

### EXAMPLES

### The Second Solution

The second solution was prepared by dissolving 120 grams of (NH₄)₂S₂O₈ in 150 ml of water at a controlled temperature of 45 °C.

### The Acid Mix

The acids mix was prepared by adding equal volumes of HCl, normality noted for each example, and H₃PO₄ (85 wt %). 0.2 g of FeCl₂.4H₂O was added to the HCl prior to contacting the HCl with the H₃PO₄.

### The Disks

The mirror polished ASTM F1537 wrought CoCrMo disk, 19.1 mm (0.75 inch) in diameter, was used in each example. Such disks are available by machine cut from a bar stock (ASTM F1537 wrought) and a mirror polished finish (Ra <0.1 µm)

### SEM Images

SEM images were obtained at 20 kV under high vacuum (FEI Company, Quanta FG 600)

### The Etching Examples

Table 1 presents a variety of experiments where HCl normality in the acid mix and the ratio of second solution to acids mix is varied. The acid mix contains HCl acid (at 6 to 9 N) and H₃PO₄ (85%). The acid mix contained 1 part (volume) of HC1 and 1 part (volume) of H₃PO₄. Second solution was prepared by dissolving 120 g ammonium persulphate in 150 ml RO water at 45 C. Weight loss and etch power are presented for each. Etch power is defined by the weight loss of the mirror polished ASTM F1537 wrought CoCrMo disk 19.1 mm (0.75 inch) in diameter per unit area exposed to acid.

**Table 1**

| Example | Etch time (min) | HCl normality | Second soln/acids mix (v/v) | Weight loss (mg) | Etch power (mg.min⁻¹) |
|---|---|---|---|---|---|
| 1 | 30 | 6 | 8/80 | 4.1 | 9.3 |
| 2 | 30 | 6 | 12/80 | 12.2 | 27.7 |
| 3 | 30 | 6 | 16/80 | 1.6 | 3.6 |
| 4 | 30 | 6 | 32/80 | 0.5 | 1.2 |
| 5 | 30 | 7 | 4/80 | 1.8 | 4.1 |
| 6 | 30 | 7 | 2/80 | 0.9 | 2.0 |
| 7 | 30 | 7 | 4/80 | 1.2 | 2.7 |
| 8 | 30 | 7 | 8/80 | 4.6 | 10.4 |
| 9 | 30 | 7 | 16/80 | 6.7 | 15.2 |
| 10 | 30 | 7 | 6/80 | 4.3 | 9.6 |
| 11 | 30 | 7 | 8/80 | 4.3 | 9.7 |
| 12 | 30 | 7 | 16/80 | 17.6 | 39.8 |
| 13 | 30 | 7 | 16/80 | 14.0 | 31.6 |
| 14 | 30 | 7 | 20/80 | 20.9 | 47.3 |
| 15 | 30 | 7 | 24/80 | 19.3 | 43.7 |
| 16 | 30 | 7 | 32/80 | 1.5 | 3.4 |
| 17 | 20 | 8 | 1/80 | 0.1 | 0.2 |
| 18 | 20 | 8 | 2/80 | 0.3 | 0.7 |
| 19 | 20 | 8 | 4/80 | 2.2 | 5.0 |
| 20 | 20 | 9 | 8/80 | 3.7 | 8.4 |

Figure 1 shows the etch power of a spectrum of etch formulations using data extracted from Table 1. These curves disclose that the highest etch power did not exist with the highest oxidant (second solution) concentration. Rather, a unique combination of the second solution/acids-mix ratio must be applied in order to achieve maximum power.

While any ratio of hydrogen chloride to persulphate that provides adequate etching can be used, in some embodiments the preferred molar ratio of hydrogen chloride to persulphate is 4:1 to 134:1. In other embodiments, the ratio is from 5:1 to 19:1.

### Example 21

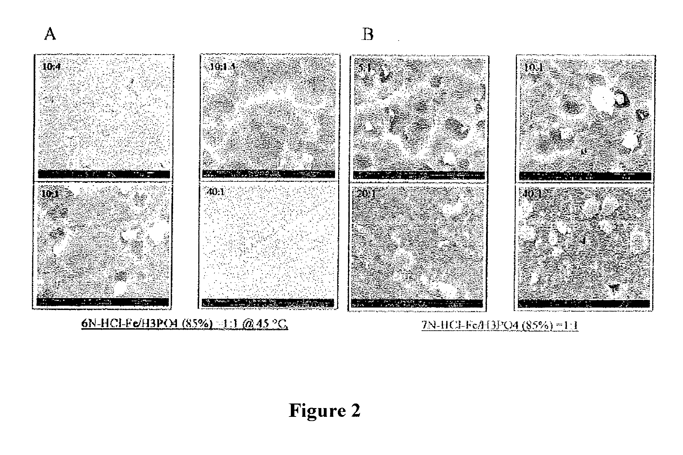

SEM images of etched ASTM E1534 wrought CoCrMo disks are shown in Figure 2. Acids-mix contains 200 ppm FeCl₂.4H₂O and 85% H₃PO₄ and HCl (6 or 7N). The HCl to H₃PO₄ ratio is 1: 1 (v/v). The second solution contains 120 g ammonium persulphate in 150 ml water at 45°C. (A) The disks were etched using 6 N HCl. The acids-mix to second solution ratio varies between 10:4 to 40:1; (B) The disks were etched using 7N HCl. The acids-mix to second solution ratio varies between 5:1 to 40: 1. Etched morphology may vary depending on acids-mix to second solution ratio.

### Example 22

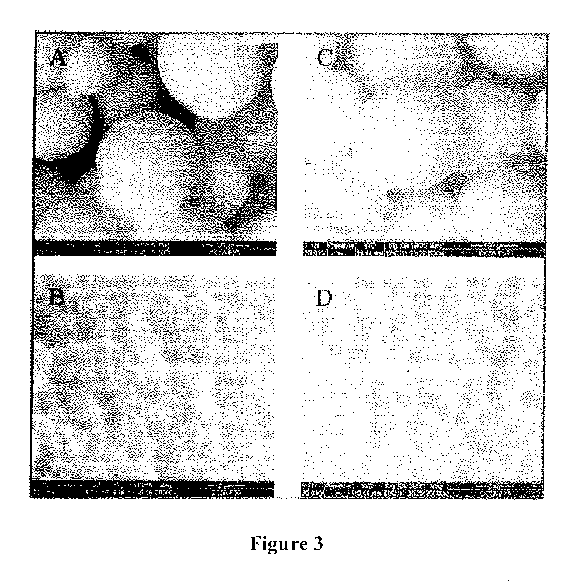

Figure 3 shows SEM images of an etched LCS knee femoral CoCrMo Porocoat (DePuy Cork) implant. The acid-mix used contained 200 ppm FeCl₂.4H₂O. 85% H₃PO₄ and HCl (6 or 7N) were used and the ratio of HCl to H₃PO₄ was 1:1 (v/v). The second solution contained 120 g of ammonium persulphate in 150 ml of water at 45 °C. In Figure 3, (A) and (B) are images of knee femoral etched with 6N HCl for 60 min at a 10: 1 acids-mix to second solution ratio (v/v). (C) & (D) are knee femoral etched with 7N HCl for 30 min at 10:2 acid-mix to second solution ratio (v/v). SEM images at 20,000x (B&D) show that the etched micron sized (0.5 to 5 µm) morphology. SEM images at 20,000x (A&C) show that the microtextured morphology is distributed evenly in 3D.

### Example 23

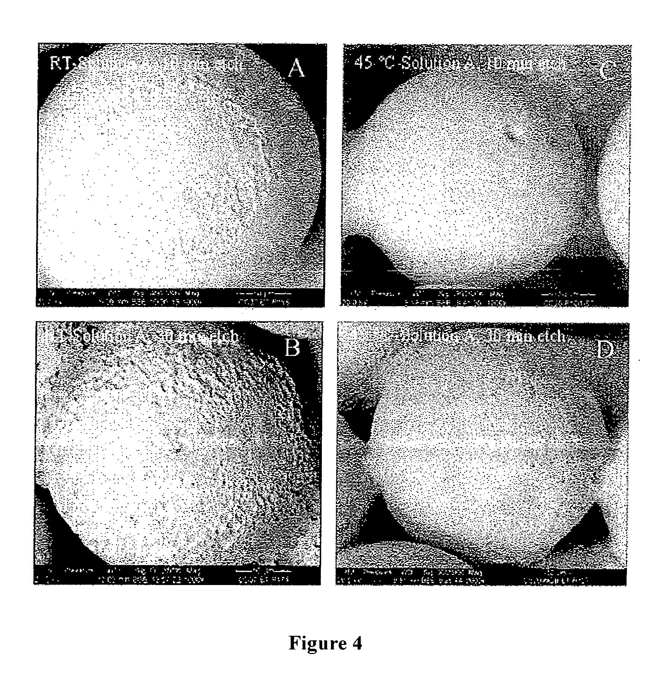

Figure 4 shows SEM images of etched knee type Porocoat (CoCrMo) using a second solution prepared with and without controlled heat input. Room temperature (RT) second solution was prepared at room condition without controlled temperature. 45°C-second solution was prepared in a temperature (heat)-controlled environment at 45°C during dissolution. (A)&(B) show that the etched morphology was uneven after etching for both 10 min and 30 min without temperature control of the second solution preparation; (C)&(D) show that the etched morphology was evenly distributed after etching for both 10 min and 30 min, with 45°C controlled second solution preparation. In this example, the second solution contained 2 g NH₄Cl and 4 g (NH₄)₂S₂O₈ and 12.5 ml H₂O. The acids-mix contained 20 mL HCl(12N), 20 ml H₃PO₄ (85 wt%).

### Example 24

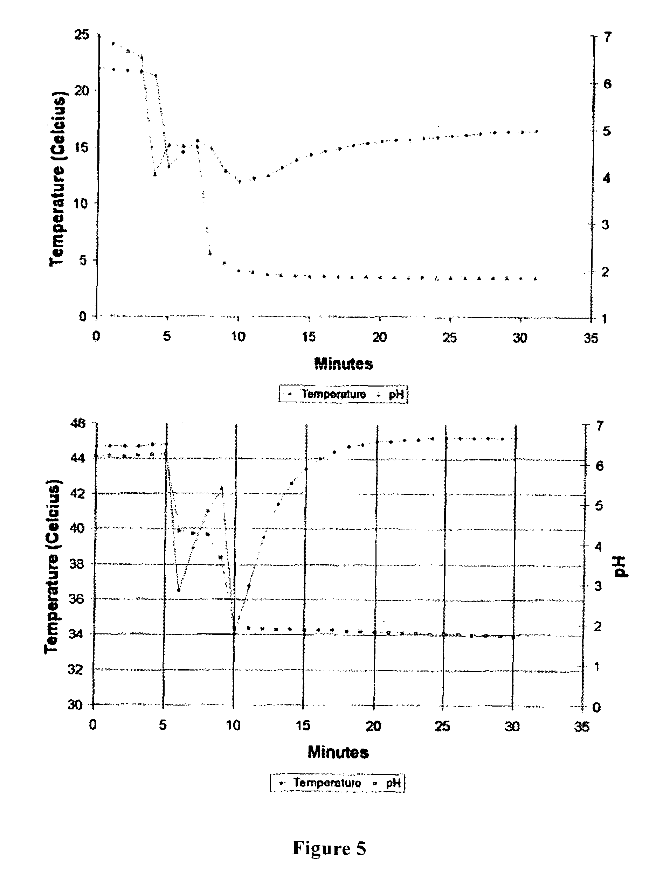

Figure 5 shows temperature and pH profiles of the second solution prepared at room condition without controlled temperature (A) and at 45°C (B). The second solution contained 2 g NH₄Cl and 4 g (NH₄)₂S₂O₈ and 12.5 ml H₂O. NH₄Cl was added first in the water followed by (NH₄)₂S₂O₈.

### Example 25 (Table 2A-C)

Results of etching metal disks are presented in Tables 2A-C.

A first solution has an HCl concentration of 6 to 8N HCl. 0.2 g of FeCl₂.4H₂O was added in 1 litre of HCl and fully dissolved by stirring. HCl concentrations of the first solution used in the example are listed in the table.

A second solution was prepared by adding ammonia persulphate (NH₄)₂S₂O₈ in 45°C water in a double glass incubator and the temperature of the second solution is maintained at 45°C before use. The concentration of the second solution used in the example is listed in the table.

The test disks were presoaked by the following process. The mirror polished wrought CoCrMo test disks (ASTM F1537), 19.1 mm (0.75 inch) diameter and 3.2 mm (0.125 inch) thick, were attached to a polytetrafluoroethylene fixture so that only one surface of the test disk is exposed when immersed in 80 ml (V1) of the first solution. The disks were immersed in the first solution for 30 minutes.

Following the presoaking, the disks were etched by adding a second solution of designated volume (V2) into the first solution and the test disks are etched for 60 minutes.
The following definitions apply to the tables:
V1: volume ofHCl solution (1st solution)
V2: volume of ammonia persulphate solution (2nd solution)
[HCl]: HCl concentration in etch solution
[APS]: ammonia persulphate concentration in etch solution
Weight loss: difference of weight of a test disk before and after etch
Etch power: weight loss per unit surface area due to etch process

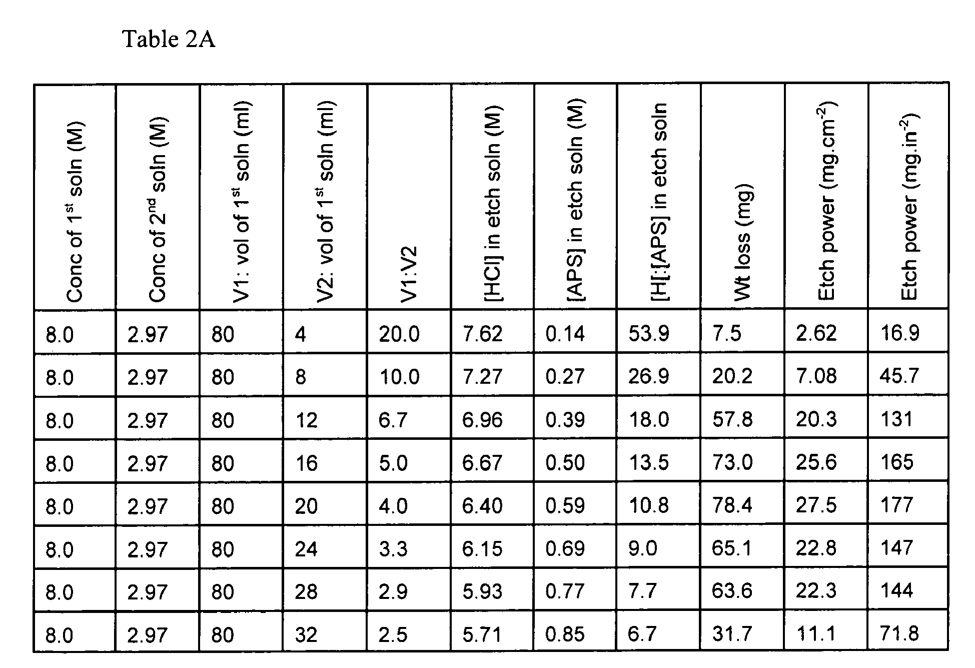

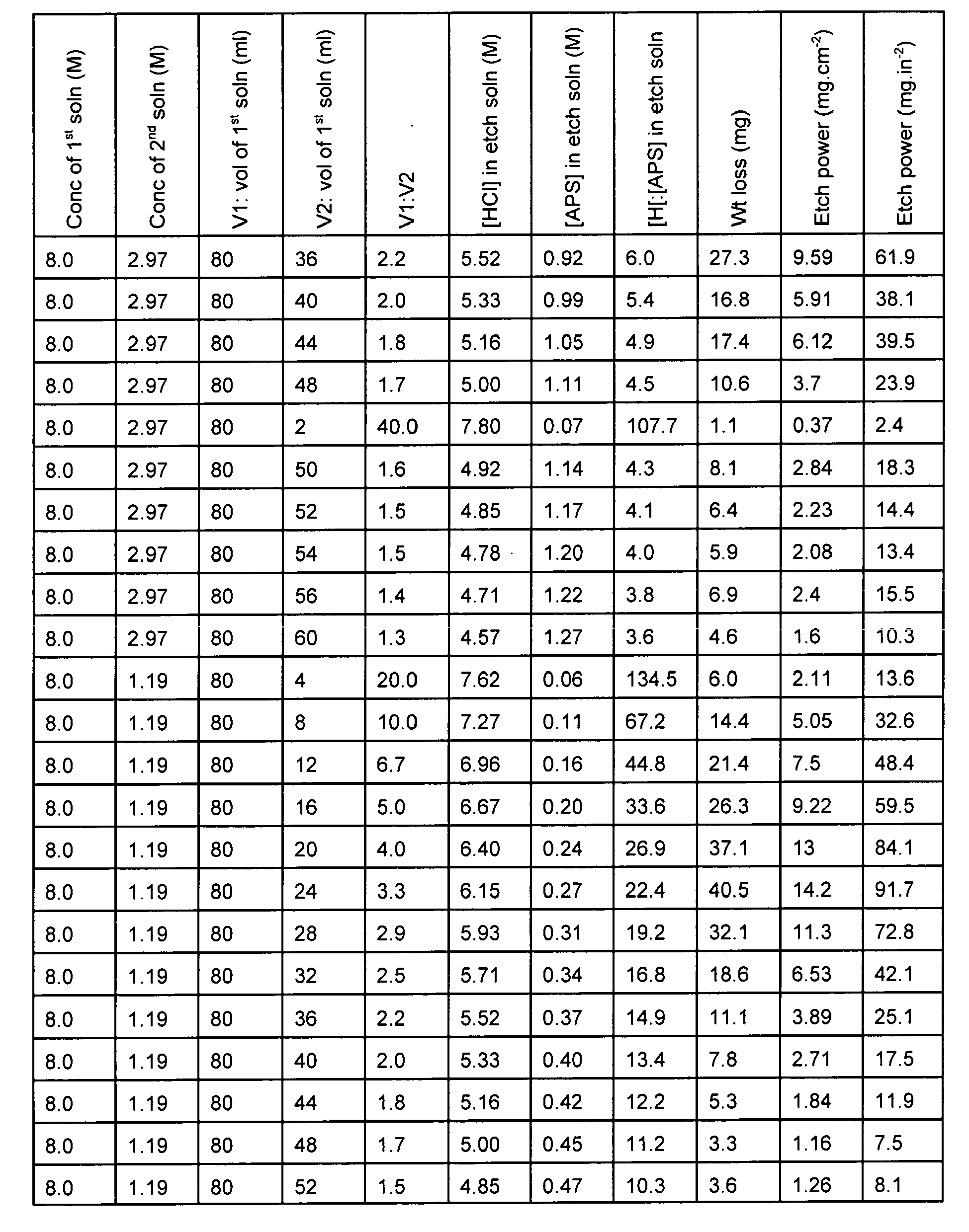

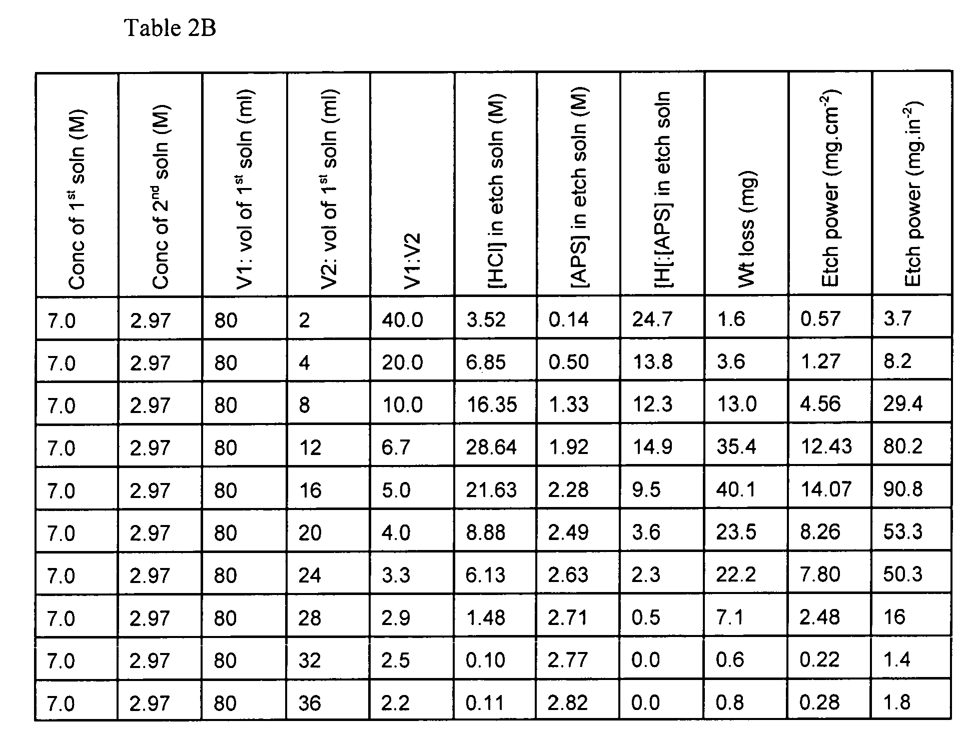

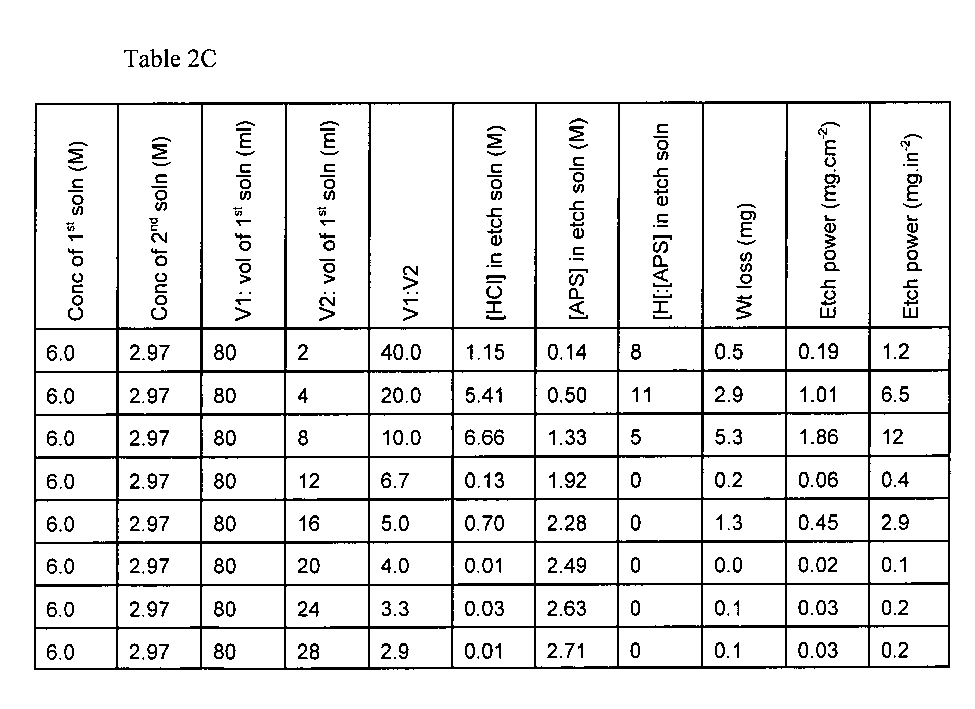

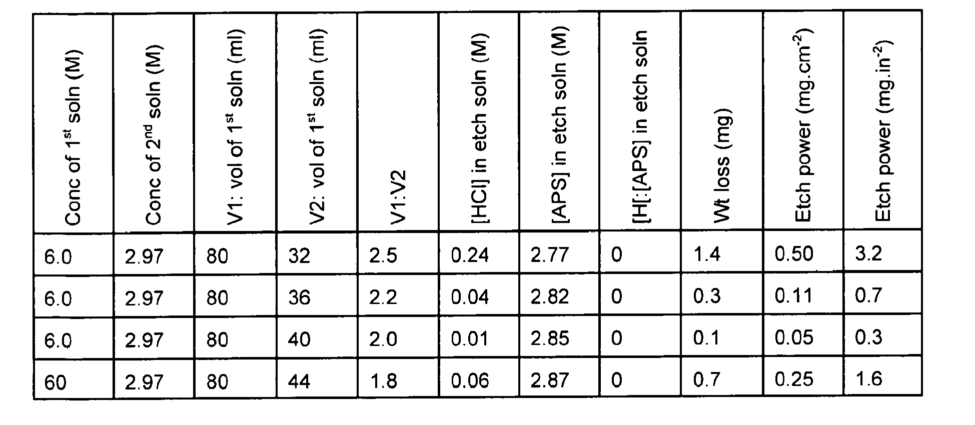

### Example 26

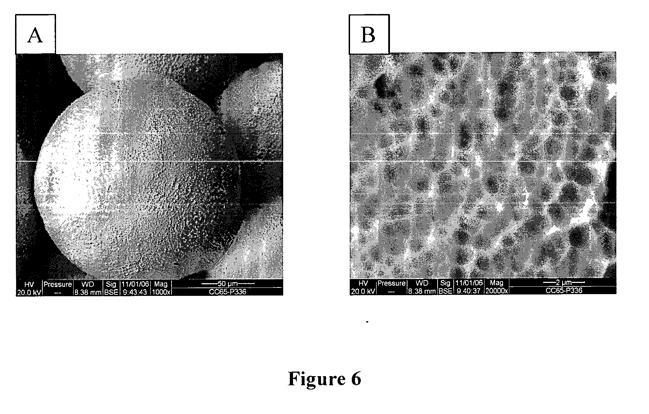

CoCrMo PFC knee Porocoat was presoaked in 80 ml 8N HCl acid for 30 minutes (containing 200 ppm FeCl₂.4H₂O) and then 4 ml of 2.97 M ammonium persulphate salt at 45 °C was added to the HCl solution. The formulation did not contain H₃PO₄. The Porocoat was etched for 60 minutes All Porocoat beads were etched, with each bead surface overlaid with craters ranging from 0.5 µm to 2 µm in sizes. (Figure 6B).

### Example 27

Acid 1 has an HCl concentration of 8N HCl. 0.2g of FeCl₂.4H₂O was added in 1 litre of HCl and fully dissolved by stirring. Acid 2 is composed of 8N HCl.

A second solution was prepared by adding ammonia persulphate (NH₄)₂S₂O₈ in 45 °C water in a double glass incubator and the temperature of the second solution is maintained at 45°C before use. The second solution contained 0.75 M ammonia persulphate.

The double sides (two large surfaces) polished wrought CoCrMo test disks (ASTM F1537), 19.1 mm (0.75 inch) diameter and 3.18 mm (0.125 inch) thick, were attached to a through hole polytetrafluoroethylene fixture so that both polished surfaces of the test disk were exposed during etch process.

Etch solution 1 was prepared by adding 20 ml of second solution in 80 ml acid 1 (contained FeCl₂). Etch solution 2 was prepared by adding 20 ml of second solution in 80 ml acid 2 (no FeCl₂).

To be processed in etch solution 1, test disks (n=2 for each time point) were pre-soaked in 80 ml acid 1 (contained FeCl₂) for zero, 5, 10 and 30 minutes before adding the second solution (ammonia persulphate). To be processed in etch solution 2, test disks (n=2 for each time point) were pre-soaked in 80 ml acid 2 (no FeCl₂) for zero, 5, 10 and 30 minutes before adding the second solution (ammonia persulphate). Table 3 shows that the etch power is higher when Fe²⁺ was present in the acid than without. Additional method to add Fe²⁺ can include immersing a Fe or Fe containing component in the acid to obtain dissolved Fe²⁺ in the acid.

**Table 3 Effect of FeCl₂ and pre-soaking time on etch power**

| EP | 0 min | 5 min | 10 min | 30 min | |
|---|---|---|---|---|---|
| Etch solution 1 | 42.8±0.2 | 46.6±1.1 | 49.0±1.3 | 43.9±0.8 | (mg.in²) |
| | 6.63 | 7.22 | 7.60 | 6.80 | (mg.cm²) |
| Etch solution 2 | 32.2±1.3 | 34.7±0.7 | 35.2±1.0 | 35.0±1.3 | (mg.in²) |
| | 4.99 | 5.38 | 5.46 | 5.43 | (mg.cm²) |

Etch solution 3 was prepared by pre-mixing acid 1 with the second solution for 5 minutes followed by adding the test disks. The test disks (n=2) were added in the etch solution 3 without previously contacting acid. Etch solution 4 was prepared by pre-mix acid 2 with the second solution for 5 minutes followed by adding the test disks. The test disks (n=2) were added in the etch solution 4 without previously contacting acid. Table 4 shows that the etch power approached zero in both cases.

**Table 4 Effect of premix on etch power (EP)**

| EP | Premix 5 min | |
|---|---|---|
| Etch solution 3 | 0.1±0.0 | (mg.in²) |
| | 0.016 | (mg.cm²) |
| Etch solution 4 | 0.1±0.1 | (mg.in²) |
| | 0.016 | (mg.cm²) |

## Claims

1. An etch solution comprising hydrogen chloride and a persulphate salt; wherein the solution has a hydrogen chloride concentration of about 3 to about 11.7 moles/litre, a molar ratio of hydrogen chloride to persulphate salt of about 4:1 to about 134:1; wherein the persulphate salt is dissolved in the solution with the use of heat input.

2. The etch solution of claim 1, which includes ferrous chloride in an amount up to 3.5 M in the etch solution.

3. The etch solution of claim 1, in which the persulphate salt is ammonium persulphate, which is preferably present in a concentration of 1 to 170 g per 100 ml of water.

4. A method comprising:
• providing a first solution comprising hydrogen chloride, in which the hydrogen chloride is present at a concentration of about 6 moles/litre to about 12 moles/litre;
• combining the first solution with a second solution comprising a persulphate salt in water to produce a third solution; in which the concentration of the persulphate salt in the second solution is 1 to 170 grams per 100 ml of water, the persulphate salt being dissolved in water at a temperature of from 10°C to 100°C, and the volume ratio of the first solution to second solution is about 3:5 to about 30:1.

5. The method of claim 4, in which the volume ratio of the first solution to the second solution is about 3:5 to about 30:1.

6. The method of claim 4, in which the persulphate salt is ammonium persulphate or potassium persulphate.

7. The method of claim 4, in which the second solution is prepared by contacting ammonium persulphate with water under heating conditions to maintain the temperature at 20°C to 70°C.

8. The method of claim 4, in which the concentration of hydrogen chloride in the first solution is from 6 to 9 moles/litre.

9. The method of claim 4, in which the first solution is formed at ambient temperature up to 100°C.

10. The method of claim 4, further comprising contacting the third solution with a metal object for a time sufficient to etch the metal; said metal object having been previously contacted with an acid solution having a proton concentration of about 1 to about 12 moles/litre.

11. The method of claim 10, in which the metal is contacted with the third solution for about 1 second to about 60 minutes.

12. The method of claim 10, in which the metal is contacted with the acid solution having the same concentration as the first solution.

13. A method of etching a metal object comprising contacting the metal object with
• an acid solution having a proton concentration of about 1 to about 12 moles/litre; and
• an etching solution; said etching solution comprising hydrogen chloride and a persulphate salt; in which the solution has a hydrogen chloride concentration of about 3 to about 11.7 moles/litre, a molar ratio of hydrogen chloride to persulphate salt of about 3:1 to about 135:1

14. The method of claim 13 in which the etching solution is made by a process comprising:
• providing a first solution comprising hydrogen chloride, in which the hydrogen chloride is present at a concentration of about 6 moles/litre to about 12 moles/litre;
• combining the first solution with a second solution comprising a persulphate salt in water to produce the etching solution; in which the concentration of the persulphate salt in the second solution is 1 to 170 grams per 100 ml of water, and the volume ratio of the first solution to second solution is about 3:5 to about 30:1.

15. A method comprising:
• contacting a metal with a solution A having a proton concentration of about 1 to about 12 mole/litre, preferably about 4 to about 10 mole/litre, and preferably containing ferrous chloride;
• contacting said metal with a solution B having a proton concentration of about 3 to about 11.7 moles/litre, a persulphate salt where the ratio of hydrogen chloride to persulphate salt of about 4:1 1 to about 134 :1, and ferrous chloride at a concentration of 20 ppm to 3.5 M,
in which the method preferably includes the step of adding the persulphate salt to solution A while the metal is in contact with solution A.
